# EUROPÄISCHE PATENTSCHRIFT

(11) **EP 2 799 110 B1**
(45) Veröffentlichungstag und Bekanntmachung des Hinweises auf die Patenterteilung: **28.02.2024**
(21) Anmeldenummer: 14001541.3
(22) Anmeldetag: 02.05.2014
(51) Int. Cl.: A61N 1/40, A61N 1/32

(54) **Medizinisches Gerät zum Erfassen und Erzeugen von Resonanzfrequenzen**
Medical device for registering and generating resonant frequencies
Dispositif médical pour l'enregistrement et la génération de fréquences de résonance

(30) Priorität: 02.05.2013 DE 102013007448
(43) Veröffentlichungstag der Anmeldung: 05.11.2014
(73) Patentinhaber: Rayonex Biomedical GmbH, 57368 Lennestadt (DE)
(72) Erfinder: Heimes, Dietmar Paul, 57368 Lennestadt (DE)
(74) Vertreter: König Szynka Tilmann von Renesse Patentanwälte Partnerschaft mbB Düsseldorf

(56) Entgegenhaltungen:
- EP-A1- 0 686 406
- EP-A1- 2 450 075
- DE-A1- 19 752 934
- DE-A1-102009 002 134
- Rayonex: "4. User Manual of the Rayocomp PS 1000 Polar Contents", , 10. August 2010 (2010-08-10), Seiten 1-99, XP055132394, Internet Gefunden im Internet: URL:http://www.extrao.fr/index.php/image/n ews/76/9000EN_FB069_4_Benutzerhandbuch_PS1 000polar_Rev3.pdf [gefunden am 2014-07-30]
- Rayonex: "Rayocomp PS 10 Portable High-Performance Bio-Resonator User Manual", , 15. September 2011 (2011-09-15), Seiten 1-72, XP055132391, Internet Gefunden im Internet: URL:http://www.extrao.fr/index.php/image/n ews/75/9100EN_FB-146_Rayocomp_PS_10_Handbu ch_Rev28.pdf [gefunden am 2014-07-30]

## Beschreibung

Die Erfindung betrifft ein medizinisches Gerät, insbesondere ein Bioresonanzgerät, zum Erfassen und Erzeugen von Resonanzfrequenzen.

Das Gerät nach der Erfindung dient der Ermittlung von Daten betreffend den physiologischen Zustand eines tierischen oder menschlichen Patienten sowie deren Auswertung im Hinblick auf den physiologischen Zustand und die mögliche Therapie des Patienten.

In den letzten Jahren hat die Bioresonanz als Diagnose- und/oder Behandlungsmethode der alternativen Medizin zunehmende Bedeutung gewonnen. Grundlage der Bioresonanztherapie ist die Suche (Analyse) und Gabe (Therapie) von Frequenzen und Frequenzspektren mit dem Ziel, Eigenregulationen bei Menschen und Tieren wieder in Gang zu setzen.

Nach der Lehre der Bioresonanz strahlt der menschliche oder tierische Körper unterschiedliche elektromagnetische Schwingungen ab. Zellen, Gewebe und Organe haben jeweils spezifische Schwingungen. Diese Einzelschwingungen stehen miteinander in Verbindung und beeinflussen sich gegenseitig. Gemeinsam bilden sie das Gesamtschwingungsspektrum des Patienten, das individuelle Schwingungsbild.

Nach der Theorie der biophysikalischen Medizin treten bei Fehlfunktionen im menschlichen oder tierischen Körper charakteristische Schwingungen auf, anhand derer sich die Natur der Störung identifizieren lässt. Diese charakteristischen Schwingungen (Frequenzen) lassen sich von außen beeinflussen, was zu einer Aufhebung des Störungszustandes kommt.

Zur Anwendung der Bioresonanztherapie sind verschiedene Vorrichtungen bekannt, so beispielsweise aus der deutschen Gebrauchsmusterschrift 32 10 955. Dabei wird ein Detektor mit dem Patienten in Kontakt gebracht, der die elektromagnetischen Schwingungen des Patienten, bzw. einzelner Körperpartien oder Organe aufnimmt und an die Bioresonanzvorrichtung überträgt. In Abhängigkeit von den aufgenommen Schwingungen werden von der Bioresonanzvorrichtung elektromagnetische Frequenzen generiert, die an den Patienten weitergeleitet werden (d.h. sie werden "aufgeprägt"). Damit wird das therapeutische Ziel erreicht.

Somit ist bei der Bioresonanztherapie die möglichst exakte, fehlerfreie Ermittlung der individuellen Schwingungen wie auch die schnelle Auswertung eine wesentliche Grundlage für eine gezielte Diagnose einer Störung und für eine wirkungsvolle Therapie mit raschem Therapieerfolg.

Die aus dem Stand der Technik bekannten Geräte nutzen dazu in der Regel die Messung des Hautwiderstandes des Patienten. Da der Hautwiderstand infolge zahlreicher Einflüsse wie Hautbeschaffenheit, Durchblutung, Umgebungstemperatur oder Wassergehalt im Körper sehr stark variiert, ist dieses Messverfahren nur eingeschränkt verwendbar, da es leicht zu falschen Messergebnissen führen kann. Mit dieser Methode kann also nicht ausreichend sichergestellt werden, dass das individuelle Schwingungsbild des Patienten und die Reaktion des Patienten auf die therapeutische Schwingung (Biofeedback) tatsächlich zutreffend erfasst werden. Diese Unsicherheit wirkt sich negativ auf den Therapieerfolg und die Aussagekraft der Diagnose aus.

Ein weiteres Bioresonanzgerät ist als Rayocomp PS 1000 Polar bekannt, siehe "4. User Manual of the Rayocomp PS 1000 Polar Contents" vom 10. August 2010 (http://www.extrao.fr/index.php/image/news/76/9000EN FB069 4 Benutzerhandbuch PS10 00polar Rev3.pdf). Dieses Gerät dient ebenfalls der "Aufprägung" bestimmter Frequenzen auf den Patienten.

In ähnlicher Weise offenbart die AT 504 713 A1 eine Vorrichtung und ein Verfahren zum Empfangen von Signalen über Sensoren (z. B. Elektroden) und Applizieren von Frequenzinformationen (über modulierte Magnetfelder und/oder über moduliertes Licht, wie bspw. Laserlicht).

Es ist jedoch mit beiden Geräten nicht möglich, Daten betreffend den physiologischen Zustand eines tierischen oder menschlichen Patienten auszuwerten und so eine mögliche Therapie des Patienten vorzuschlagen.

Aufgabe der Erfindung ist es, ein demgegenüber verbessertes System für die Anwendung der Bioresonanzdiagnostik und -therapie bereitzustellen.

Diese Aufgabe wird gemäß der Erfindung durch die Bereitstellung eines Geräts gemäß Anspruch 1 gelöst.

Das Gerät ist dabei für dessen Steuerung umfassend die folgenden Schritte ausgestaltet:
a) Ermitteln von elektrophysiologischen Datenpunkten eines menschlichen oder tierischen Patienten in Abhängigkeit von auf diesen Patienten (Mensch oder Tier) aufgeprägten elektromagnetischen Frequenzen,
b) Vergleichen dieser elektrophysiologischen Datenpunkte mit einem Standardwert für diese elektrophysiologische Datenpunkte,
c) Identifizieren mindestens einer der Frequenzen aus Schritt a), bei der der elektrophysiologische Datenpunkt von dem Standardwert abweicht (nachfolgend auch "Resonanzfrequenz" oder "Interferenzwert") und
d) Abgleichen der mindestens einen Resonanzfrequenz des Patienten mit einer Datenbank umfassend vorbekannte Resonanzfrequenzmuster.

Der Erfindung liegt die Erkenntnis zugrunde, dass ein menschlicher oder tierischer Organismus auf einem Stimulus in Form einer auf ihn einwirkenden ("aufgeprägten") elektromagnetischen Frequenz physiologisch reagiert und die Summe möglicher verschiedener Reaktionen als Veränderung eines elektrophysiologischen Signals des Organismus erkennbar ist. Die Veränderung des elektrophysiologischen Signals ist messbar. Je nach physiologischem Ausgangszustand des Organismus ist die Veränderung des elektrophysiologischen Messwerts bei verschiedenen Stimuli, hier also Frequenzen, besonders erkennbar. Demnach kann das in Abhängigkeit von verschiedenen Frequenzen erfasste Reaktionsmuster aus elektrophysiologischen Messwerten des Organismus zur Diagnose von Krankheits- oder Belastungszuständen des Organismus genutzt werden.

Die Erfinder haben demnach festgestellt, dass aufgrund der Abweichung eines elektrophysiologischen Messwerten von einem Standardwert Resonanzfrequenzen ("Resonanzfrequenzmuster") eines menschlichen oder tierischen Patienten ermittelt werden können, die geeignet sind, Auskunft über den physiologischen Zustand dieses Patienten zu geben. Nach dem Verfahren können für den Vergleich entweder diese Messwerte als solche, oder aber aus diesen Messwerten berechnete Werte herangezogen. Sofern nicht ausdrücklich anders angegeben, wird demnach zusammenfassend von elektrophysiologischen "Daten" oder "Datenpunkten" gesprochen. Die aus den Messwerten berechneten Werte werden im Rahmen dieser Anmeldung als "rechnerisch verarbeitete Datenpunkte" bezeichnet. Somit werden von den Begriffen "Daten" oder "Datenpunkte" sowohl die elektrophysiologischen Messwerte umfasst, als auch die daraus berechneten sogenannten "rechnerisch verarbeiteten Datenpunkte".

Die mit Hilfe des Verfahrens erstellten individuellen Resonanzfrequenzmuster können außerdem zu therapeutischen Zwecken genutzt werden. So können z.B. mit Hilfe eines Bioresonanzgeräts Frequenzen auf den Patienten aufgeprägt werden, die dem Störungszustand entgegenwirken und so den Patienten möglichst wieder in seinen physiologischen Normalzustand (d.h. gesunden Zustand) bringen.

Das erfindungsgemäße Gerät ist für ein Verfahren ausgestaltet. Nach diesem Verfahren werden, wie ausgeführt, von dem Patienten zunächst elektrophysiologische Messwerte erhoben. Diese Messwerte sind vorzugsweise Ergebnisse eines Verfahrens, das aus der Gruppe der folgenden Verfahren ausgewählt ist: Elektroenzephalographie (EEG), Elektrokardiographie (EKG), Elektrogastrographie, Elektrokochleographie, Elektronystagmographie, Elektrookulographie, Elektroretinographie, Elektromyographie, und Elektroneurographie. Bevorzugt sind Messwerte aus der Elektroenzephalographie und der Elektrokardiographie. Besonders bevorzugt wird das Verfahren mit Messwerten aus der Elektrokardiographie durchgeführt.

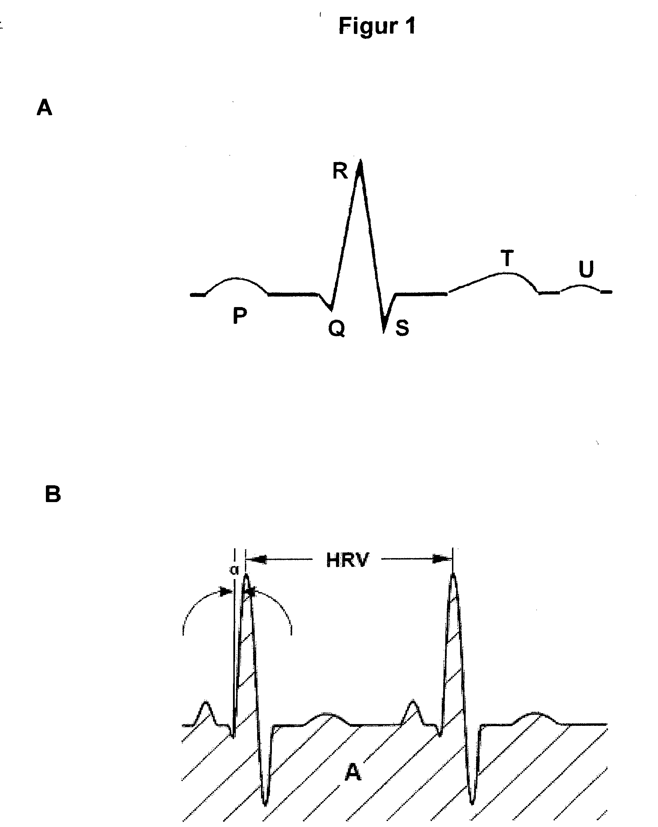

All diesen Verfahren ist gemein, dass die elektrische Aktivität, d.h. die Leitung eines elektrischen Impulses bzw. einer Erregung, eines Organs oder Gewebes erfasst wird. Diese Aktivität wird über die Zeit gemessen und in für das jeweilige Verfahren typischen Kurvenverlaufen erfasst. Die erfindungsgemäß relevanten Messwerte werden demnach als Messwertkurven dargestellt. Die Gestalt der jeweiligen Kurve, z.B. die Lage und Steilheit bestimmter Peaks, gibt dem medizinischen Fachmann Anhaltspunkte über den physiologischen Zustand des jeweiligen Organs bzw. Gewebes. Bei einem EKG wird zum Beispiel ein Herzschlag in einer typischen Kurve dargestellt, die als P-Q-R-S-T-U Kurve bekannt ist. Die einzelnen Buchstaben sind Bezeichnungen für charakteristische Peaks, Täler oder sonstige typische Merkmale der Kurve (siehe dazu auch Abb. 1A).

In dem Verfahren werden die vom Patienten ermittelten elektrophysiologischen Messwerte, also die entsprechenden Messwertkurven, vorzugsweise nicht unmittelbar (d.h. im Sinne von Rohdaten) für den Vergleich mit Standardwerten oder Standardkurven eingesetzt, sondern werden zunächst rechnerisch verarbeitet ("verrechneter Datenpunkt"). In einer besonders vorteilhaften Ausführungsform der Erfindung wird die Fläche unter einer Kurve (Area Under the Curve; AUC) erfasst und als Ausgangspunkt für den Vergleich mit einem Standardwert herangezogen. Dem Fachmann sind die Methoden zur Berechnung der Fläche unter einer Kurve bekannt. Für diese Berechnung wird zunächst ein für das jeweilige Verfahren sinnvoller Kurvenabschnitt definiert. Zum Beispiel wird bei einem EKG als Kurvenabschnitt vorzugsweise eine vollständige P-Q-R-S-T-U Kurve, d.h. also die Erregungsleitung eines Herzschlags definiert. In diesem Fall wird nach dem Verfahren demnach die jeweilige Fläche unter einer bei einer bestimmten Frequenz gemessenen P-Q-R-S-T-U Kurve als bevorzugter elektrophysiologischer Datenpunkt ermittelt. Vorzugsweise werden mindestens zwei oder mehr solcher Datenpunkte ermittelt, d.h. es wird eine Mehrzahl von Flächen ermittelt. Die Kurven werden in Abhängigkeit von zeitgleich aufgeprägten Frequenzen aufgenommen.

Alternativ oder ergänzend können zu der Flächenberechnung andere rechnerisch zu ermittelnde Charakteristika der vom Patienten erfassten elektrophysiologischen Messwerte (Kurven) zur Darstellung und Analyse des Zustands des Patienten herangezogen werden. In der besonders bevorzugten Ausführungsform auf Basis eines EKG wird vorzugsweise die Steilheit des Q-R-S Peaks ("Gradient") ausgewertet. Dieser kann durch einen Winkel alpha beschrieben werden, der dem Winkel zwischen der Senkrechten zur X-Achse (= Zeitachse im EKG) und dem ansteigendem Schenkel der R-Zacke entspricht (siehe Abb. 1B). In einer besonders bevorzugten Ausführungsform wird sowohl der Winkel alpha als auch die Fläche unter der Kurve berechnet. Auch bei anderen elektrophysiologischen Messwerten bzw. Kurven kann die Steilheit bestimmter Kurvenabschnitte in die Auswertung einfließen oder aber die Auswertung kann darauf beruhen.

Alternativ oder ergänzend können zu den bereits genannten Messwerten Gradient und Fläche unter der Kurve (AUC) auch ein oder mehrere der kardiovaskulären Messwerte ausgewählt aus der Gruppe umfassend Herzfrequenz, Herzrhythmus, Länge, Amplitude oder Form der P-Welle, der T-Welle, der U-Welle oder des QRS-Komplexes, Länge des PQ-Intervalls, des QT-Intervalls, des RR-Intervalls oder der ST-Strecke herangezogen werden. Ganz besonders bevorzugt wird die Herzfrequenz, insbesondere bevorzugt die Herzfrequenzvariabilität (engl. Heart rate variability; HRV) herangezogen. Diese kann auf Basis von drei bis vier Herzschlägen bestimmt werden. Eine Steuerung auf Basis von 3-4 Herzschlägen ist vorteilhaft, da die HRV nur kurzzeitig auf die von außen aufgeprägten Frequenzen reagiert und sich nach spätestens 3-4 Herzschlägen wieder auf die neue Situation anpasst.

Die elektrophysiologischen Messwerte des Patienten werden nach dem Verfahren in Abhängigkeit von auf den Patienten aufgeprägten elektromagnetischen Schwingungen (Frequenzen) erfasst. Das bedeutet, dass die Messwerte erfasst werden, während der Patient die elektrischen Impulse, d.h. die Stimulation, durch die aufgeprägten Frequenzen erhält. Damit wird Schritt a) des Verfahrens umgesetzt. Der Patient ist also zeitgleich mit einer Vorrichtung zur Erfassung der elektrophysiologischen Messwerte und mit einer Vorrichtung zur Aufprägung (Stimulation) der Frequenzen auf den Patienten verbunden. Beide Vorrichtungen können getrennt oder kombiniert sein.

Auf den Patienten werden erfindungsgemäß in einem ersten Schritt mindestens zwei, insbesondere mehr als zwei Frequenzen aufgeprägt. In einer Ausführungsform werden die mehr als zwei Frequenzen als Abfolge von Einzelfrequenzen aufgeprägt, d.h. die Frequenzen wirken nacheinander und nicht als Gemisch verschiedener Frequenzen auf den Patienten ein. So kann die Wirkung einer bestimmten Einzelfrequenz auf den Patienten ermittelt werden.

In einer Ausführungsform des Verfahrens werden zwischen 10 bis 500, bevorzugt zwischen 50 und 400, weiter bevorzugt zwischen 100 und 200 Frequenzen auf den Patienten geprägt. Damit erfolgt dann eine sog. Bereichswertmessung. Bei jeder dieser Frequenzen wird eine elektrophysiologische Messwertkurve aufgenommen. Bei N aufgeprägten Frequenzen werden somit auch N elektrophysiologische Messwertkurven aufgenommen.

Die aufgeprägten Frequenzen liegen vorzugsweise im Bereich von 0,1 Hz bis 10,0 GHz. In einer bevorzugten Ausführungsform liegen sie im Bereich von 0,1 Hz bis 100 MHz und bevorzugt im Bereich von 0,1 Hz bis 500 MHz.

Sofern die Frequenzen sequenziell, d.h. in einer Abfolge, aufgeprägt werden, können sie zueinander denselben Frequenzunterschied aufweisen, d.h. die Frequenzfolge besitzt eine einheitliche Schrittweite. Wenn beispielsweise eine Abfolge von Frequenzen mit einem Frequenzwert von 0,5 kHz; 1,0 kHz; 1,5 kHz; 2,0 kHz; 2,5 kHz; 3,0 kHz; 3,5 kHz; 4,0 kHz etc. verwendet werden, beträgt die Schrittweite 0,5 kHz.

In einer Ausführungsform werden jeweils einzelne Frequenzen auf der Patienten aufgeprägt. Es ist aber bevorzugt, jeweils Frequenzspektren aufzuprägen, d.h. mehrere Einzelfrequenzen gleichzeitig dem Patienten zu applizieren.

Vorzugsweise handelt es sich bei den aufgeprägten Spektren um dekadische Frequenzspektren, d.h. um Spektren, die jeweils von einer Einzelfrequenz als Grundfrequenz ausgehen, und dann nur weitere Frequenzen aufweisen, die jeweils um den Faktor 10 gegenüber der jeweils niedrigen Frequenz erhöht sind. Der niedrigste Frequenzwert wird als "Frequenzgrundwert" bezeichnet. So würde bei einem Frequenzgrundwert von 0,5 kHz das dekadische Frequenzspektrum Frequenzen von 5 kHz, 50 kHz, 500 kHz usw. enthalten. Ein dekadisches Frequenzspektrum enthält ausschließlich solche jeweils um den Faktor 10 voneinander abweichende Frequenzen.

In dieser bevorzugten Ausführungsform steht somit der Frequenzgrundwert nicht für eine konkrete Einzelfrequenz, sondern für den kleinsten Wert eines Frequenzspektrums, wobei die weiteren Frequenzen jeweils eine um den Faktor 10ⁿ höhere Frequenz aufweisen. Sofern nicht näher spezifiziert, wird im Kontext der vorliegenden Erfindung unter dem allgemeinen Begriff Frequenz sowohl die Einzelfrequenz als auch ein Frequenzspektrum verstanden.

In einer bevorzugten Ausführungsform wird zunächst die niedrigste Frequenz aufgeprägt.

Dann wird schrittweise der Frequenzbereich bis zur höchsten Frequenz durchlaufen. Die Abfolge kann aber auch genau anders herum sein, d.h. es kann auch mit der höchsten Frequenz begonnen werden und dann eine schrittweise Erniedrigung bis zur kleinsten Frequenz erfolgen.

Gemäß Schritt b) des Verfahrens werden die elektrophysiologischen Datenpunkte mit einem Standardwert für diese Datenpunkte verglichen, um so mindestens eine Resonanzfrequenz zu ermitteln. Die Resonanzfrequenz ist diejenige Frequenz, dessen elektrophysiologischer Datenpunkt vom Standardwert abweicht. Bei der Aufprägung einer Mehrzahl von Frequenzen (Bereichswertmessung) können zwei oder mehr Resonanzfrequenzen gefunden werden. Häufig liegt die Anzahl von ermittelten Resonanzfrequenzen zwischen 10 und 100, vorzugsweise zwischen 50 und 80 Resonanzfrequenzen. Die Anzahl der bei einer Bereichswertmessung ermittelten Resonanzfrequenzen kann durch die Verwendung eines Filters beeinflussbar sein.

Als Standardwert kann ein Wert aus einer externen Datenbank herangezogen werden, d.h. ein Wert, der nicht auf den individuellen Patienten zurückgeht. So sind, wie oben ausgeführt, die typischen Kurvenverläufe der erfindungsgemäßen elektrophysiologischen Messwerte dem Fachmann grundsätzlich bekannt. Die vom Patienten bei einer bestimmten Frequenz ermittelte Kurve kann mit einer solchen typischen Kurve verglichen werden. Als Vergleichsmaßstab kann dazu vorzugsweise die Analyse der Fläche unter der Kurve dienen.

In einer bevorzugten Ausführungsform wird ein Standardwert herangezogen der in der jeweiligen Bereichswertmessung des Patienten ermittelt wird. Das Heranziehen des Mittelwertes aus der patienteneigenen Bereichswertmessung als Sollwert hat den Vorteil, dass sich darin der individuelle elektrophysiologisch erfassbare Zustand des Patienten zum Zeitpunkt der Messung widerspiegelt.

Der patienteneigene, d.h. individuelle, Standardwert ist vorzugsweise der Mittelwert aller für den Patienten ermittelten Datenpunkte im Verlaufe einer Messung. Dieser Standardwert kann auch als Sollwert bezeichnet werden. Auch für diese Berechnung wird vorzugsweise die Fläche unter der Messwertkurve herangezogen. Diejenigen Frequenzen, bei denen die Fläche der Messwertkurve vom Mittelwert aller erfassten Flächen abweicht, stellen dann die gesuchten Resonanzfrequenzen dar. Das Maß der Abweichung, das als diagnostisch oder therapeutisch relevant erachtet wird, kann vom Fachmann bestimmt werden. Durch die Ermittlung der Resonanzfrequenzen entsteht ein individueller "energetischer Fingerabdruck" des Patienten.

In einer bevorzugten Ausführungsform wird zusätzlich erfasst, ob sich der Messwert durch die aufgeprägte Frequenz erhöht oder erniedrigt. Diese Wirkung wird durch die sog. Polarität der Resonanzfrequenz zum Ausdruck gebracht ("+" für Erhöhung; "-". für Erniedrigung).

Für die Auswertung der ermittelten Resonanzfrequenzen durch den Abgleich mit einer Datenbank mit vorbekannten Resonanzfrequenzmustern (Schritt d) des Verfahrens) können diese verschiedenen Auswahlkriterien mit Hilfe entsprechender Filter genauer dargestellt und ausgewertet werden. Die Resonanzfrequenzmuster aus der Datenbank repräsentieren vorzugsweise bestimmte physiologische Zustände. Den Datenbanken liegen eine hohe Anzahl verschiedener Bereichswertmessungen unterschiedlicher Patienten zugrunde. Die erfindungsgemäß heranzuziehenden Datenbanken geben somit Standardresonanzfrequenzmuster wieder. In der Datenbank sind vorzugsweise Resonanzmuster sowohl für einen gesunden Normalzustand als auch für pathologische Zustände abgelegt. In einer Ausführungsform der Erfindung umfasst die Datenbank mehr als 500, bevorzugt mehr als 1500 Standardfrequenzmuster.

Der Abgleich der individuellen Resonanzfrequenzen mit den Standardresonanzfrequenzmustern aus einer Datenbank ermöglicht die Ermittlung des physiologisches Zustandes des Patienten.

In einer Ausführungsform werden die durch das Verfahren ermittelten Resonanzfrequenzen dazu genutzt, für diesen Patienten therapeutisch wirksame Frequenzen auszuwählen. Die Auswahl kann manuell durch den Therapeuten erfolgen oder aber automatisiert durch das Gerät, insbesondere durch ein Bioresonanzgerät. Die Auswahl der Frequenzen folgt vorteilhafterweise nach dem Prinzip des "similia similibus curentur", d.h. es werden dem Patienten zur Therapie ("Harmonisierung") seiner Störung identische oder ähnliche Frequenzen aufgeprägt.

In diesem Aspekt kann das Gerät, insbesondere ein Bioresonanzgerät, für ein Verfahren mit folgenden Schritten ausgestaltet sein:
a) Ermitteln von elektrophysiologischen Datenpunkten eines menschlichen oder tierischen Patienten in Abhängigkeit von auf diesen Patienten (Mensch oder Tier) aufgeprägten elektromagnetischen Frequenzen,
b) Vergleichen dieser elektrophysiologischen Datenpunkte mit einem Standardwert für diese elektrophysiologischen Datenpunkte,
c) Identifizieren mindestens einer der Frequenzen aus Schritt a), bei der ein elektrophysiologischer Datenpunkt von dem Standardwert abweicht ("Resonanzfrequenz" oder "Interferenzwert") und
d) Abgleichen der mindestens einen Resonanzfrequenz des Patienten mit einer Datenbank umfassend Resonanzfrequenzen und Identifizieren mindestens eines Resonanzfrequenzmusters,
e) Erzeugen der mindestens einen nach c) identifizierten Resonanzfrequenz und/oder des mindestens einen nach d) identifizierten Resonanzfrequenzmusters und
f) Übermitteln der Resonanzfrequenz und/oder des Resonanzfrequenzmusters nach e) auf mindestens eine mit dem Gerät verbundene Elektrode.

In einer besonders vorteilhaften Ausgestaltung kann das Verfahren zum Betrieb des medizinischen Geräts als ein Regelkreislauf gestaltet sein, indem der Standardwert als Sollwert und die nach Schritt a) erhobenen Datenpunkte den Istwert darstellen. Nach dieser Ausführungsform können die nach den Schritten e) und f) erzeugten und übermittelten Resonanzfrequenzen auf den Patienten aufgeprägt und dessen elektrophysiologischen Datenpunkte gemäß Schritt a) in Abhängigkeit von diesen Frequenzen ermittelt werden. Das Verfahren wird dann so oft iterativ durchlaufen, bis es keine medizinisch relevante Abweichung mehr gibt zwischen den Istwerten und dem Sollwert.

In einem weiteren Aspekt kann das Verfahren die folgenden Schritte umfassen:
a) Erfassung mindestens eines kardiovaskulären Messwertes (Istwert) eines Patienten in Abhängigkeit von auf diesen Patienten aufgeprägten elektromagnetischen Frequenzen,
b) Vergleich des kardiovaskulären Messwerts mit einem Sollwert
c) Steuerung eines Moduls zur Erzeugung elektromagnetischer Frequenzen (Frequenzgeneratormodul) in Abhängigkeit von der Differenz zwischen Messwert und Sollwert.

So haben die Erfinder gefunden, dass die zielgerichtete Steuerung eines Bioresonanzgerätes in sehr vorteilhafter Weise durch die Erfassung und Auswertung kardiovaskulärer Messwerte erfolgen kann.

Diese Ausführungsform hat insbesondere den Vorteil, dass kardiovaskuläre Daten von äußeren, für die Therapie unmaßgeblichen Reizen weniger beeinflussbar sind und so zu einem zuverlässigeren Therapie- und Diagnoseerfolg führen. Außerdem erlauben kardiovaskuläre Messungen im Gegensatz zu Hautwiderstandsmessungen ein breites Spektrum an möglichen Auswerteparametern (Herzrate, Herzfrequenz, Größe, Zeitdauer und Form der einzelnen Wellen etc.), die in Abhängigkeit von der zu therapierenden oder diagnostizierenden Erkrankung gewählt werden können. In einer besonderen Ausführungsform wird die Fläche unter der Messwertkurve zur Auswertung herangezogen.

Die möglichen kardiovaskulären Messparameter sind durch die langjährige intensive Forschung in ihrer Bedeutung und ihrer individuellen Variabilität sehr genau bekannt und stellen somit eine gute Grundlage für ein darauf aufbauendes Steuerungssystem dar.

Diese Ausführungsform kann vorteilhafterweise als ein Regelkreis aufgebaut werden. Indem Frequenzspektren, die durch das Bioresonanzgerät auf den Patienten einwirken, ihrerseits kardiovaskuläre Veränderungen hervorrufen können, können diese Veränderungen im Sinne eines Biofeedback-Mechanismus wieder zur Anpassung der von dem Generatormodul erzeugten elektromagnetischen Frequenzen verwendet werden. Vorzugsweise werden demnach die Schritte a), b) und c) mindestens einmal, besonders bevorzugt mehrmalig wiederholt.

Der Betrieb des Bioresonanzgerätes wird in dieser Ausführungsform also über einen Regelkreis gesteuert, in dem mindestens ein kardiovaskuläres Signal als Regelgröße eingesetzt wird.

Damit erlaubt diese Ausführungsform in vorteilhafter Weise eine interaktive Veränderung der auf den Patienten einwirkenden Frequenzspektren. Wird ein Frequenzspektrum therapeutisch als wirksam detektiert, kann während der Messung direkt durch die Software entschieden werden, welches Frequenzspektrum vorteilhafterweise auf den Patienten aufgeprägt werden kann. Dies erlaubt eine effektivere und vor allem detailliertere und schnellere Analyse in Verbindung mit einer zugleich verkürzten Therapiedauer.

Der besondere Vorteil dieser Ausführungsform ist demnach, dass die Auswahl der therapeutischen Frequenz und/oder die Dauer ihrer Anwendung nicht mehr auf Erfahrungswerten basiert, sondern auf Grundlage mindestens eines am Patienten erhobenen kardiovaskulären Parameters, der nachweislich durch eine zuvor aufgeprägte Frequenz beeinflusst wurde.

In einer bevorzugten Ausführungsform geben die kardiovaskulären Messwerte die elektrischen Aktivitäten der Herzmuskelfasern wieder, wie sie beispielsweise durch ein Elektrokardiogramm (EKG) erfasst werden.

In einer Ausführungsform werden die kardiovaskulären Messwerte während der Anwendung des Bioresonanzgerätes erfasst. Diese direkte Kopplung zwischen der Erfassung der Messwerte und einer darauf abgestimmten Steuerung der elektromagnetischen Frequenzen erlaubt die Durchführung des Verfahrens in einem Regelkreis.

In einer weiteren Ausführungsform beträgt bei dem Verfahren die Zeitspanne zwischen dem Eintreffen des kardiovaskulären Messwertes in dem Bioresonanzgerät und der Erzeugung einer dadurch gesteuerten elektromagnetischen Frequenz weniger als 60 Sekunden, bevorzugt weniger als 20 Sekunden, weiter bevorzugt weniger als 5 Sekunden und besonders bevorzugt weniger als 2 Sekunden. Dies wird vorteilhafterweise durch die Verwendung einer algorithmenbasierten Software gewährleistet, die eine dynamische Reaktion auf das jeweilige Messergebnis mit kurzen Auswertezeiten ermöglicht. Die Software kann so ausgelegt sein, dass sie die exakte Peak-Bestimmung der kardialen Messkurve erlaubt. Die Auswertung der kardiovaskulären Parameter kann nach üblichen, dem Fachmann bekannten Methoden erfolgen. So kann der bevorzugte Parameter Herzfrequenzvariabilität (HRV) zum Beispiel im Zeitbereich, im Frequenzbereich oder im nichtlinearen Bereich bestimmt werden (s. Definition der HRV).

In einer Ausführungsform sind die kardiovaskulären Messwerte ausgewählt aus der Gruppe umfassend Herzfrequenz, Herzrhythmus, Länge, Amplitude oder Form der P-Welle, der T-Welle, der U-Welle oder des QRS-Komplexes, Länge des PQ-Intervalls, des QT-Intervalls, des RR-Intervalls oder der ST-Strecke. Wie oben bereits ausgeführt, stellt die Fläche unter der Kurve ein besonders relevantes Merkmal dar. Sie fließt demnach in einer bevorzugten Ausführungsform als Datenpunkt in die Auswertung ein

In einer bevorzugten Ausführungsform wird zur Steuerung die Herzfrequenz und hierbei bevorzugt die Herzfrequenzvariabilität (engl. Heart rate variability; HRV) herangezogen, die in besonders bevorzugtem Maße auf Basis von 3-4 Herzschlägen bestimmt wird. Eine Steuerung auf Basis von 3-4 Herzschlägen ist vorteilhaft, da die HRV nur kurzzeitig auf die von außen aufgeprägten Frequenzen reagiert und sich nach spätestens 3-4 Herzschlägen wieder auf die neue Situation anpasst.

Da die Herzratenvariabilität ihren Ursprung in der Funktion des vegetativen Nervensystems hat, lassen sich damit erfindungsgemäß beispielsweise Erkrankungen diagnostizieren und/oder behandeln, bei denen das autonome Nervensystem eine zentrale Rolle spielt. Dabei sind Erkrankungen zu unterscheiden, die direkt das autonome Nervensystem schädigen, und Krankheiten, die sich etwa über dauerhaft erhöhte Stoffwechselbeanspruchungen indirekt auf das autonome Nervensystem auswirken.

Ein Beispiel für eine Erkrankung mit direkter Schädigung des autonomen Nervensystems ist die diabetische Neuropathie, wohingegen beispielsweise die koronare Herzkrankheit sich indirekt auf das autonome Nervensystem auswirkt. Auch psychische Erkrankungen können über eine Erhöhung des Katecholaminspiegels und die Sympathikusaktivierung erkennbare Folgen auf die Herzaktivität haben; die Herzfrequenzvariabilität kann daher für die erfindungsgemäße Bioresonanzvorrichtung auch im Bereich der Neuropsychiatrie zu diagnostischen und therapeutischen Zwecken herangezogen werden. Auch diese Erkrankungen sind daher erfindungsgemäß behandelbar.

Weitere Erkrankungen, die erfindungsgemäß über Veränderungen der Herzratenvariabilität therapierbar sind, sind: Asphyxie bei Neugeborenen, plötzlicher Herztod nach Herzinfarkt (als prädiktiver Wert), Asthma oder Sepsis.

In einer vorteilhaften Ausführungsform wird bei dem Verfahren durch das Frequenzgeneratormodul eine Frequenz oder aber ein Frequenzspektrum aus mindestens zwei, bevorzugt zwei bis 8000 und besonders bevorzugt zwei bis 500 Frequenzen erzeugt.

In einer bevorzugten Ausführungsform wendet das Frequenzgeneratormodul hierbei die Dipol-Technologie an. Diese hat den Vorteil, dass auch weit höhere Frequenzen als 100 kHz erzeugt werden können.

Bei dem Verfahren ist das von dem Frequenzgeneratormodul erzeugte Frequenzspektrum ein dekadisches Frequenzspektrum. Unter einem dekadischen Frequenzspektrum wird im Sinne der Erfindung ein Frequenzspektrum verstanden, bei denen die Einzelfrequenzen sich in ihrer Frequenz um den Faktor 10 oder einem Vielfachen hiervon unterscheiden. In einer besonders bevorzugten Ausführungsform unterscheiden sich bei dem dekadischen Frequenzspektrum alle Einzelfrequenzen um den Faktor 10, wie beispielsweise durch das folgende Frequenzspektrum dargestellt:
- 80 kHz, 800 kHz, 8 MHz, 80 MHz, 800 MHz.

In einer besonders bevorzugten Ausführungsform wird das Verfahren iterativ durchgeführt, d.h. dass die von dem Frequenzgeneratormodul erzeugten, auf den Patienten einwirkenden elektromagnetischen Frequenzen direkt oder indirekt Auswirkungen auf die nachfolgend ermittelten kardiovaskulären Messwerte haben können.

In einem weiteren Aspekt kann das Verfahren mit einem System durchgeführt werden, das die folgenden Module umfasst:
- Modul zur Aufnahme und Auswertung mindestens eines kardiovaskulären Messwerts,
- Modul zur Erzeugung elektromagnetischer Frequenzen (Frequenzgeneratormodul) sowie
- Modul zur Steuerung des Frequenzgeneratormoduls.

In einer Ausführungsform weist das System zusätzlich eine Eingabeeinheit auf, die bevorzugt eine Tastatur und bevorzugt eine Folientastatur ist.

In einer weiteren Ausführungsform weist das System zusätzlich eine Eingabe/AusgabeEinheit auf, die bevorzugt ein Bildschirm mit einer Folientastatur und besonders bevorzugt ein Berührungsbildschirm ist.

In einer weiteren Ausführungsform weist das System eine Messvorrichtung zur Erfassung mindestens eines kardiovaskulären Messwerts auf, die bevorzugt Messsonden umfasst.

In einer Ausführungsform weist das System eine oder mehrere Elektroden auf, die zur Übermittlung der erzeugten elektromagnetischen Frequenzen auf den Patienten dienen.

In einer besonderen Ausführungsform kann das System zur Therapie oder Diagnostik von Erkrankungen verwendet werden.

Ein Bioresonanzgerät ist eine Vorrichtung zur Behandlung (Diagnose, Therapie (einschließlich Prävention)) einer Erkrankung, die elektromagnetische Schwingungen von einem Patienten aufnehmen und elektromagnetische Schwingungen auf den Patienten abstrahlen kann ("aufgeprägte Frequenz"). Bevorzugt liegen die Frequenzen im Hz, kHz oder MHz-Bereich, wobei bevorzugt Frequenzspektren bestehend aus unterschiedlichen Einzelfrequenzen verwendet werden.

Ein kardiovaskulärer Messwert ist als ein Messwert definiert, der das Herz oder das Gefäßsystem betrifft. Dies umfasst beispielsweise die Aufzeichnung der elektrischen Aktivitäten der Herzmuskelfasern, wie sie in summarischer Form durch das EKG wiedergegeben wird. Darüber hinaus fallen darunter auch Parameter wie der Blutdruck oder der (transkutan gemessene) Sauerstoffpartialdruck.

Ein "Frequenzgeneratormodul" ist als eine Vorrichtung definiert, die elektromagnetische Frequenzen erzeugt. In bevorzugter Form kann das Frequenzgeneratormodul Frequenzspektren mit unterschiedlichen Einzelfrequenzen erzeugen. Vorzugsweise lässt sich das Frequenzgeneratormodul regeln.

Unter einer Messsonde wird jede Vorrichtung verstanden, die geeignet ist, kardiovaskuläre Messwerte bei einem Patienten zu erfassen. Dies umfasst bspw. eine Messelektrode, einen Clip, der am Finger, Zeh oder Ohr angebracht werden kann (insbesondere für die Pulsoxymetrie), eine (komprimierbare) Manschette, einen Brustgürtel oder eine Pulsuhr.

Unter "Herzfrequenzvariabilität" werden die quantifizierten Schwankungen der Herzfrequenz verstanden. Synonym hierzu werden die Begriffe "Herzratenvariabilität" oder "Herzschlagvariabilität" verwendet. Der Abstand zwischen zwei Herzschlägen wird meistens definiert als die Zeit zwischen dem Beginn zweier Kontraktionen der Herzkammern. Dieser Beginn der Kammerkontraktion erscheint im Elektrokardiogramm (EKG) als so genannte R-Zacke. Der Abstand zwischen zwei R-Zacken wird daher als RR-Intervall oder auch als NN-Intervall bezeichnet. Das RR-Intervall lässt sich als Kehrwert in die Herzfrequenz umrechnen (60 BPM - 1000 ms: 60 Beats per minute - 1000 Millisekunden RR-Abstand). Die Schwankungen in der Dauer der RR-Intervalle geben somit die Herzfrequenzvariabilität wieder. Eine einfache statistische Größe zur Bestimmung der Streuung ist die Standardabweichung der RR-Intervalle. Man unterscheidet insgesamt drei Bereiche (Domänen), die zur Analyse der Herzfrequenzvariabilität genutzt werden: Den Zeitbereich (z. B. Standardabweichung der RR-Intervalle), den Frequenzbereich (z. B. Spektrum der Herzfrequenzvariabilität) und den nichtlinearer Bereich (z. B. Poincaré-Plots). Hinsichtlich ihrer Zeitskala lassen sich die Schwankungen der Herzfrequenz durch Verfahren der Spektralanalyse näher charakterisieren. In jüngerer Zeit werden auch komplexe empirische Parameter, wie z. B. die fraktale Dimension herangezogen. Die Spektralanalyse ist ein sehr genaues Verfahren zur Feststellung der Frequenzanteile, aus denen sich die Variabilität der Herzfrequenz zusammensetzt. Sie gibt beispielsweise Auskunft über die Kopplung von Atmung und Herzschlag (also deren Kohärenz) im entspannten Zustand. Sind Atmung und Herzschlag gut gekoppelt, ergibt die Spektralanalyse einen eindeutigen Peak (Spitzenwert). Das betreffende Mess-Spektrum wird in der HRV-Forschung in drei Frequenzbänder aufgeteilt, VLF (very low frequency), LF (low frequency, mitunter auch als MF (middle frequency) bezeichnet) und HF (high frequency), teilweise zuzüglich eines vierten Frequenzbandes: ULF (ultra low frequency). Hierbei repräsentiert die VLF die peripher-zentrale Thermoregulation, die LF die Oszillationen des Baroreflexes und die HF die respiratorische Sinusarrhythmie.

In einem weiteren Aspekt kann das medizinische Gerät zum Erfassen und Erzeugen von Resonanzfrequenzen die folgenden Module umfassen:
a) ein Modul zur Aufnahme elektrophysiologischer Messwerte am menschlichen oder tierischen Patienten,
b) ein Speichermodul umfassend eine Datenbank von Resonanzfrequenzen,
c) ein Modul zum Erzeugen elektromagnetischer Frequenzen sowie
d) mindestens eine Elektrode, die sowohl der Erfassung der elektrophysiologischen Messwerte als auch der Aufprägung der elektromagnetischen Frequenzen dient.

Bei dieser Ausführungsform der Erfindung werden demnach Elektroden verwendet, die sowohl zur Erfassung der elektrophysiologischen Messwerte als auch zur Aufprägung der elektromagnetischen Frequenzen dienen. Die Elektroden haben demnach eine Doppelfunktion.

Im Folgenden wird die Erfindung anhand von Beispielen erläutert. Diese Erläuterungen sind lediglich beispielhaft und schränken die Breite der Erfindung wie im unabhängigen Anspruch definiert nicht ein.

### BEISPIELE:

### Beispiel 1: Apparative Umsetzung des Verfahrens

Im Folgenden wird die Durchführung des Verfahrens anhand des Bioresonanzgeräts "Rayocomp PS 1000 polar" der Firma Rayonex Biomedical GmbH (Lennestadt, Deutschland) beschrieben.

### 1. Schritt: Auswählen des Messbereichs

Bei dem Verfahren kann auch die Polarität der aufzuprägenden Frequenzen verändert werden. Da die Frequenzen bei dem Bioresonanzgerät mit der sogenannten Dipol-Technologie erzeugt werden, handelt es sich hierbei um zirkular polarisierte Schwingungen, die als linksrotierende oder rechtsrotierende Schwingung oder in einer Überlagerung beider Schwingungsrichtungen zur Anwendung kommen können.

Im ersten Schritt kann der Messbereich und die Schrittweite der aufzuprägenden Frequenzspektren eingestellt werden. Durch Einstellung eines Startfrequenzgrundwertes von 0,0 und einem Endfrequenzgrundwert von 100,0 ergeben sich bei einer Schrittweite von 0,50 insgesamt 200 zu messende Frequenzspektren, basierend auf diesen Frequenzgrundwerten. Alternativ kann die Schrittweite beispielsweise auf 0,25 eingestellt werden und führt dann zu 400 Messkurven

### 2. Schritt: Anlegen der Elektroden

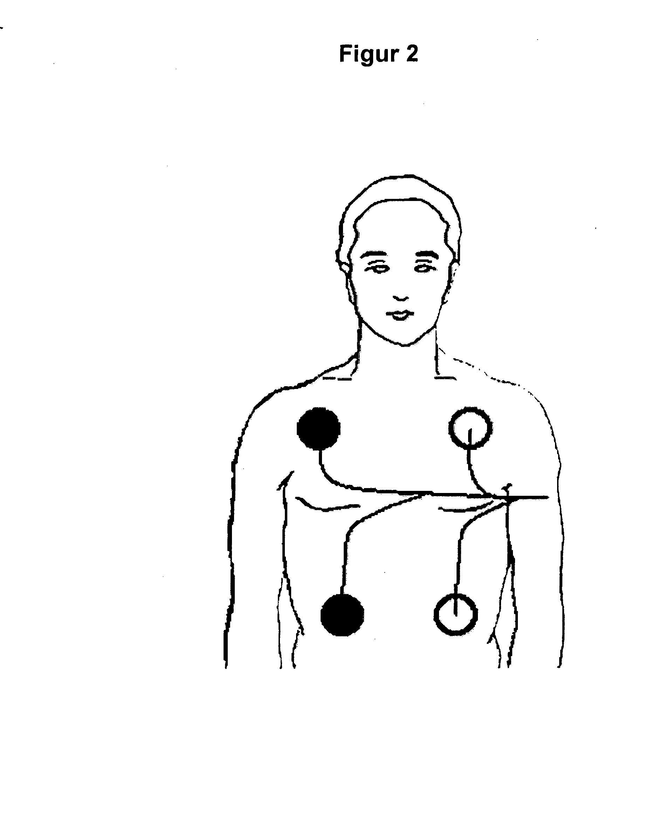

Im zweiten Schritt werden vier Elektroden am Oberkörper der zu testenden Person angebracht (s. Fig. 2) und über Kabel mit dem Bioresonanzgerät verbunden. Die Elektroden erfüllen zwei Funktionen: zum einen dienen sie zur Ankopplung der vom Bioresonanzgerät kommenden Frequenzen, zum anderen dem Messen der Herzsignale als elektrokardiologische Messwerte. Das Resonanzgerät kann auch noch über weitere Elektroden mit dem Patienten verbunden werden Diese sollten dann als Flächendetektoren oder aber als vier 30 cm langen Klettdetektoren (an Armen und Beinen) ausgestaltet sein.

### 3. Schritt: Prüfen der Signalqualität

In diesem Schritt wird die Herzsignalqualität geprüft. Es wird geprüft, ob die Verbindung der Elektroden zum Körper zur Auswerteelektronik im Bioresonanzgerät sicher und ungestört ist.

### 4. Schritt: Prüfen der besten Ableitung

Im vorherigen Schritt wurde geprüft, ob eine genügend hohe Signalqualität vorliegt. In diesem Schritt wird nun automatisch die beste Ableitung gemessen, berechnet und bewertet. Das Bioresonanzgerät ermittelt, zwischen welchen der vier Anschlüssen die beste Herzkurve zu messen ist. Die Messung kann auf einem geräteseitigen Monitor verfolgt werden, der die aktuell überprüfte Ableitung anzeigt. Diejenige Ableitung, die sich in mehreren Messdurchläufen als die beste Ableitung herausstellt, wird von dem Gerät als zur Messung geeignet vorgeschlagen.

Für den Fall, dass die Steilheit einer Kurve oder die Fläche unter der Kurve zur Berechnung der Messwerte verwendet werden soll, ist darauf zu achten, dass in dem gemessenen Elektrokardiogramm die R-Zacke als Peak nach oben zeigt.

### 5. Schritt: Die Erfassung der elektrophysiologischen Messwerte

Nun erfolgt die eigentliche Messung der auf den Frequenzgrundwerten basierenden Spektren. Das Bioresonanzgerät beginnt gemäß den gewählten Einstellungen die Frequenzgrundwerte einzustellen. Die Frequenzen werden über die Elektroden auf den Körper der zu messenden Person übertragen. Währenddessen misst das Bioresonanzgerät die Herzkurve und bewertet Veränderungen, die durch den Stimulus der aufgeprägten Frequenzen entstehen.

Die Herzkurve wird dabei auf dem Monitor des Bioresonanzgerätes dargestellt und kann über die Messung hinweg verfolgt werden. Zudem wird auf dem Monitor die im letzten Schritt bestimmte Ableitung der Herzkurve ebenso dargestellt wie der aktuelle Puls. Während der Messung hat sich der Patient ruhig zu verhalten (keine zu großen Bewegungen, normale Atmung, etc.). Wurden alle Frequenzen des Bereichs gemessen, geht das Bioresonanzgerät automatisch in die Berechnung und damit in den nächsten Schritt über.

### 6. Schritt: Berechnung und Auswahl der Resonanzfrequenzen

In diesem Schritt werden die elektrophysiologischen Messwerte mit einem entsprechendem Standardwert verglichen, und diejenigen Frequenzgrundwerte, bei denen der elektrophysiologische Messwert von dem Standardwert abweicht, als Resonanzfrequenz (auch als "Interferenzwert", "Resonanzwert" oder "Resonanzstelle" genannt) identifiziert. In bevorzugter Weise erfolgt hierbei der Vergleich auf Basis von rechnerisch bearbeiteten Datenpunkten, beispielsweise die Fläche unter der P-Q-R-S-T-U-Kurve, dem Anstieg der R-Zacke (quantifizierbar durch den Winkel alpha) oder der Herzratenvariabilität, wobei diese Datenpunkte dann mit den entsprechenden Standardwerten der jeweiligen Datenpunkte verglichen werden.

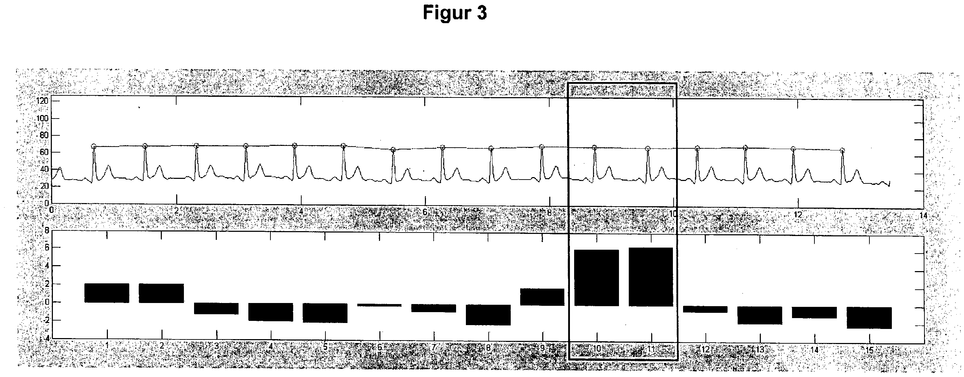

In der Figur 3 sind Daten aus der Anwendung des Verfahrens an einem humanen Patienten wiedergegeben. In dem oberen Teil der Grafik ist die durch die Messelektronik ermittelte Herzkurve dargestellt. In dem unteren Teil der Grafik ist die Differenz zwischen Standardwert und rechnerisch verarbeitetem Datenpunkt aufgetragen. Dabei ergeben sich im Vergleich vom Standardwert Schwankungen nach oben oder nach unten. Wird der Patient mit einem Stimulus beaufschlagt, der ihn nicht tangiert, d.h. den er nicht mit Energie verarbeiten muss, so verbleibt der Wert in der normalen Schwankungsbreite. Gibt man aber auf den Körper einen Stimulus durch das Bioresonanzgerät, auf den der Körper reagieren muss (hier ein Stimulus durch das Bioresonanzgerät nach Paul Schmidt=BnPS in der durch den Rahmen dargestellten Zeitspanne), dann zeigt sich in den Messungen eine über die normale Schwankungsbreite hinausgehende Veränderung des Datenpunktes (hier eine Beschleunigung der Herzrate).

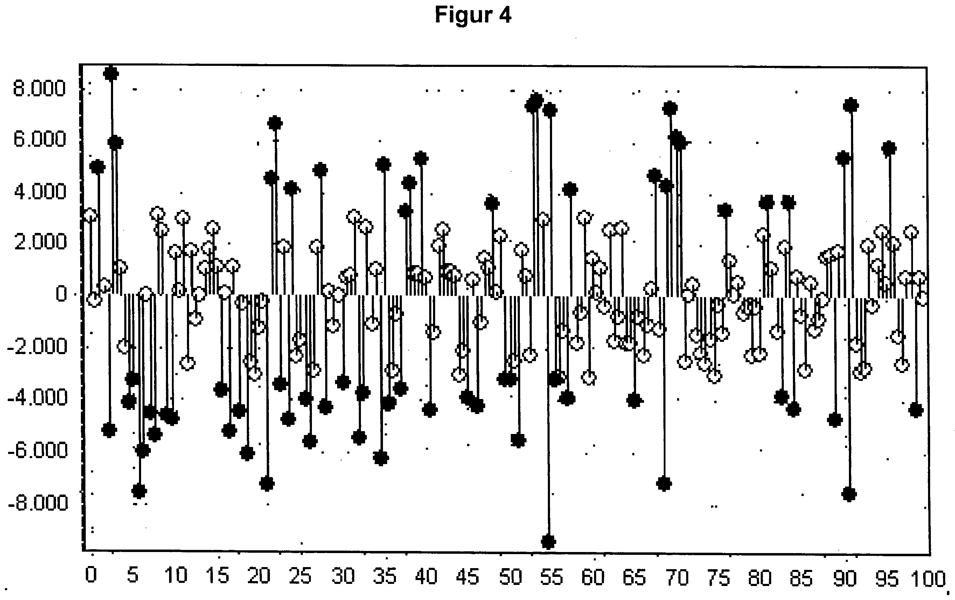

Des Weiteren werden in diesem Schritt die auffälligen Resonanzfrequenzen von dem Gerät in einer grafisch aufbereiten Form angezeigt (siehe Figur 4). Sowohl Frequenzgrundwerte, von denen eine tonisierende (d.h. verstärkende) Wirkung als auch eine sedierende (d.h. abschwächende) Wirkung der Herzaktivität ausging, sind für die Diagnose als auch die Therapie interessant und werden automatisch mit der bipolaren Funktion +/- harmonisiert.

In diesem Beispiel werden von dem Gerät von 200 Messwerten ca. 68 der am stärksten wirkenden Resonanzfrequenzen angezeigt. Die Anzahl dieser priorisierten Sequenzen kann durch eine Verstellung der Signifikanzschwelle verändert werden.

Weiterhin können durch manuelle Eingabe auch die 20 stärksten Resonanzfrequenzgrundwerte ausgewählt werden. Die jeweils markierten Resonanzfrequenzen werden nach Bestätigung des Anwenders übernommen und können so im letzten Schritt weiter bearbeitet und analysiert werden.

### 7. Schritt: Ergebnis und Berechnung der Therapie-Programme

Im letzten Schritt werden die gewonnen Resonanzfrequenzen weiter bearbeitet. Diese können nach ihrer Polarität, nach ansteigendem Frequenzgrundwert oder der Stärke der durch sie erzeugten Veränderung sortiert werden. Die letztgenannte Sortierung ist hierbei am sinnvollsten.

In der Speicherverwaltung lassen sich die einzelnen Frequenzgrundwerte weiter analysieren.

Durch einen Vergleich der identifizierten Resonanzfrequenzen mit in einer Datenbank abgespeicherten Frequenzmustern, kann ermittelt werden, welche Frequenzmuster besonders wichtig für den Patienten sind. In dem beispielhaften Bioresonanzgerät sind mehr als 1700 Therapie-Programme abgespeichert. Die aufgefundenen Resonanzfrequenzen bilden somit eine Art "energetischer Fingerabdruck", der all jene Frequenzgrundwerte beschreibt, die der Patient aktuell für seine Regulation benötigt.

Die Resonanzwerte können zudem in der jeweils gewählten Sortierreihenfolge auf eine Speicherkarte abspeichert werden.

### Beispiel 2: Anwendungsbeispiel - Patient A

Ein Patient wurde mit dem Gerät "Rayocomp PS 1000 polar" (Fa. Rayonex, Lennestadt, Deutschland) anhand der im Beispiel 1 geschilderten Schritte untersucht.

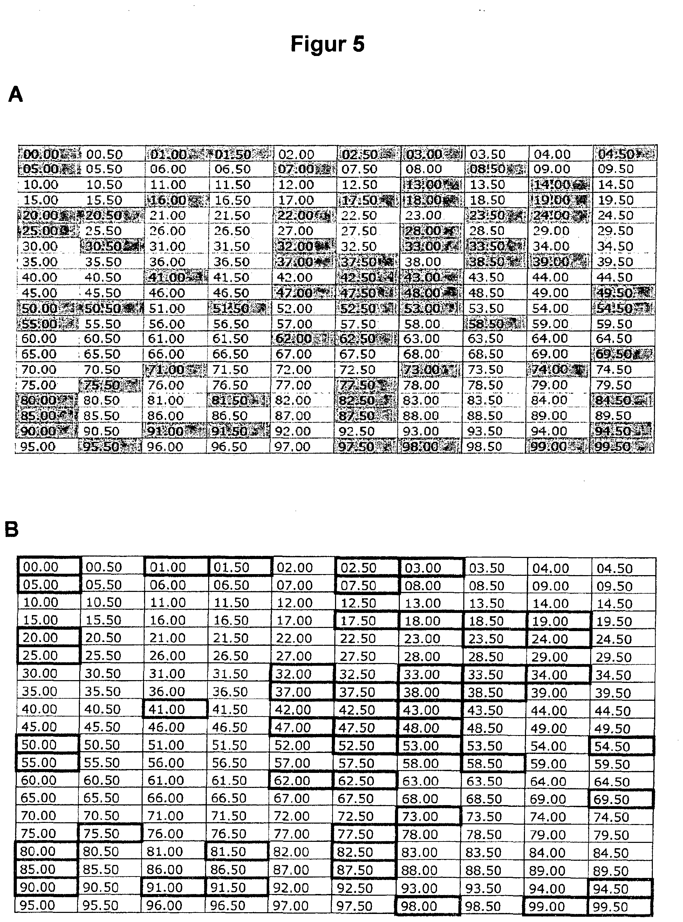

Die Aufprägung von insgesamt 200 Frequenzen zwischen 0 kHz und 99.5 kHz ergab insgesamt 68 Resonanzfrequenzen, die in der Figur 5A grau unterlegt dargestellt sind.

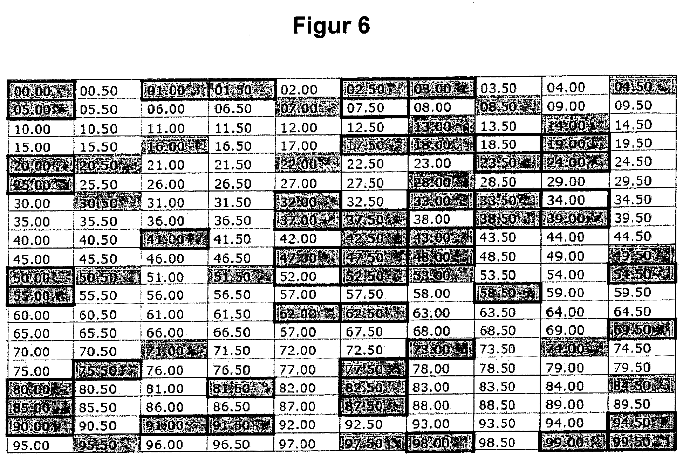

In der Figur 5B ist das in der Datenbank abgelegte Frequenzmuster für eine Gallenblasenentzündung mit insgesamt 52 Frequenzgrundwerten dargestellt. Wie in Figur 6 dargestellt, ergab der Vergleich dieses abgespeicherten Musters mit den ermittelten Resonanzfrequenzen eine Übereinstimmung von 89%, weist also auf eine entsprechende Erkrankung hin.

### Beispiel 3: Anwendungsbeispiel - Patient B

Eine Patientin (56 Jahre, weiblich) wurde mit dem Gerät "Rayocomp PS 1000 polar" (Fa. Rayonex Biomedical GmbH, Lennestadt, Deutschland) anhand der im Beispiel 1 geschilderten Schritte untersucht.

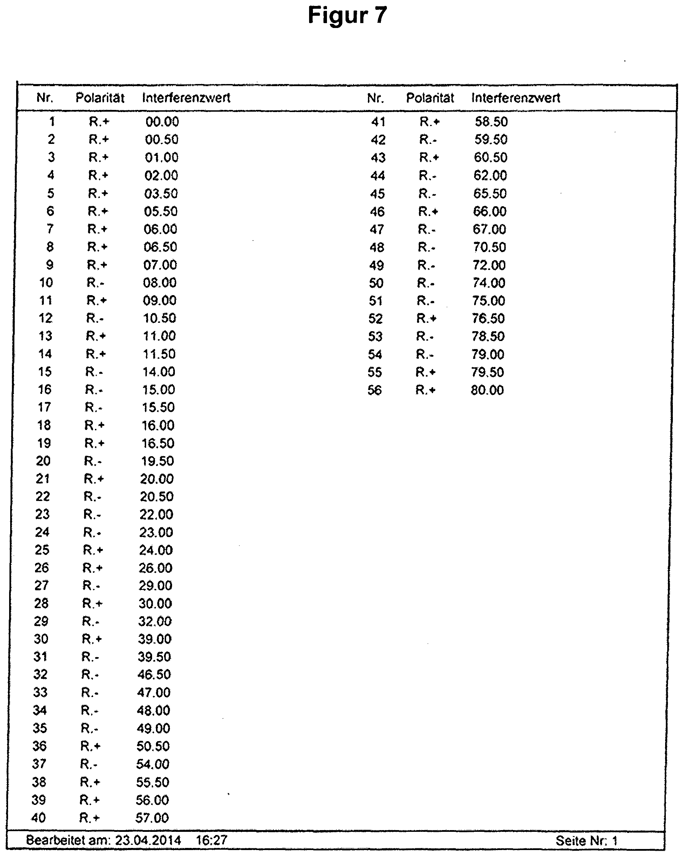

Zuerst wurden die Resonanzfrequenzgrundwerte - wie im Beispiel 1 - mit dem Bioresonanzgerät ermittelt. Die ermittelten 56 Resonanzfrequenzgrundwerte sind in Figur 7 aufgelistet.

Diese Resonanzfrequenzgrundwerte (Interferenzwerte) stellen all jene Frequenzen dar, die der Organismus der Patientin aktuell für seine Regulation benötigt. Im vorliegenden Fall wurden 56 Resonanzfrequenzgrundwerte ermittelt.

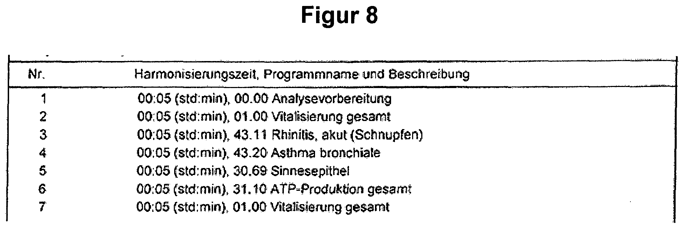

Anschließend wurden über 1700 Therapieprogrammen mit dem Muster der Resonanzfrequenzgrundwerte der Patientin verglichen. Das Ergebnis dieses Vergleichs ist in Figur 8 wiedergegeben.

Hierbei ist zu beachten, dass geräteseitig als erstes, zweites Programm sowie als letztes Programm Standardprogramme aufgelistet werden, und somit also nur die übrigen Therapieprogramme spezifisch auf den Zustand des Patienten beziehen.

Als erstes fand das System das Programm mit der Nummer 43.11 Rhinitis, dann das Programm mit der Nummer 43.20 Asthma bronchiale. Das Bioresonanzgerät schlägt also diese Therapieprogramme als die beiden wichtigsten Therapieprogramme für diese Patientin vor.

Auf der Basis der erfindungsgemäßen Messung wurden dann sowohl die Resonanzfrequenzgrundwerte als auch die Therapie-Programme auf eine Speicherkarte für die anschließende Therapie der Patientin gespeichert und bereitgestellt.

### Beispiel 4: Aufbau eines erfindungsgemäßen Bioresonanzgerätes

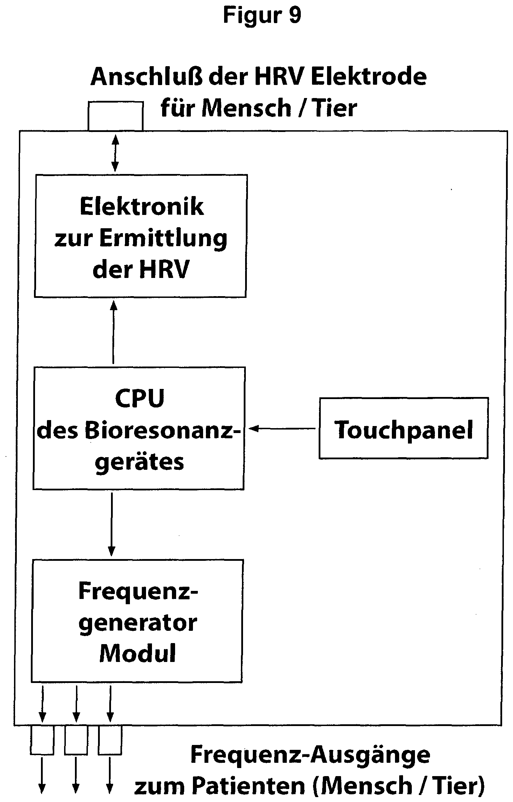

Wie in der Figur 9 schematisch dargestellt, weist das erfindungsgemäße Bioresonanzgerät die folgenden Bestandteile auf: Durch einen Anschluss für Elektroden, die die kardiovaskulären Messwerte, insbesondere die Herzfrequenz, erfassen, werden diese Messwerte in das Gerät eingespeist. Innerhalb des Gerätes werden die Messwerte in einer elektronischen Datenverarbeitungseinheit zur Bestimmung der Herzratenvariabilität ausgewertet und an den Hauptprozessor (CPU) weitergeleitet. In dem Hauptprozessor erfolgt der Vergleich der erhobenen Herzfrequenzvariabilität mit einem Sollwert, der als Schwellenwert vorgegeben ist. Oberhalb dieses Schwellenwertes wird die Steuerung des Frequenzgeneratormoduls entsprechend verändert. Das vom Frequenzgeneratormodul erzeugte Spektrum an elektromagnetischen Frequenzen wird über Frequenzausgänge und daran befestigte Elektroden an den Patienten übermittelt. Zur externen Steuerung des Bioresonanzgerätes besitzt es ein Touchpanel.

### Beispiel 5: Darstellung des Regelkreises

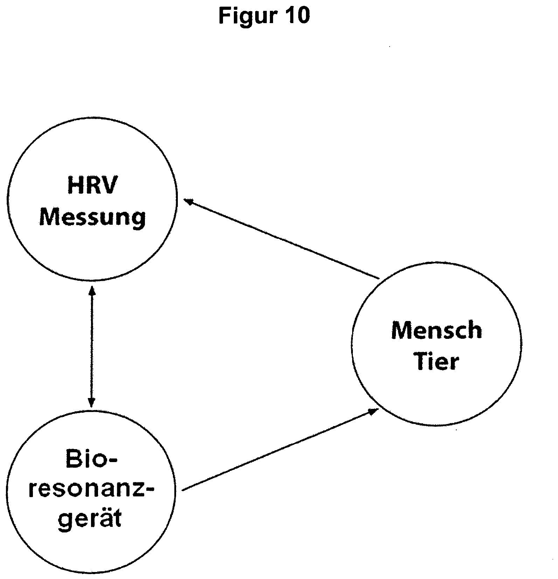

In Figur 10 ist schematisch der Regelkreis dargestellt, wie er sich durch die Anwendung des Verfahrens ergibt. Hierbei wirkt das Bioresonanzgerät auf den Menschen oder das Tier als Patienten ein. Bei dem Patienten wird die daraus resultierende Variabilität der Herzfrequenz mittels einer Messsonde gemessen und wieder in das Bioresonanzgerät eingespeist, welches in Abhängigkeit von der gemessen Herzratenvariabilität das erzeugte Frequenzspektrum beibehält oder entsprechend einem vorgegebenen Algorithmus abändert. Im Sinne eines technischen Regelkreises ist hierbei das erzeugte elektromagnetische Frequenzspektrum als Regelstrecke aufzufassen, die Herzfrequenzvariabilität als Regelgröße und die CPU des Bioresonanzgerätes als Regler.

### Abbildungslegenden:

- Fig. 1A:: Schematische Darstellung einer EKG-Kurve mit Zuordnung der einzelnen Abschnitte als P-Welle (P), negativer Q-Ausschlag (Q), R-Zacke (R), negativer S-Ausschlag (S), T-Welle (T) und U-Welle.

- Fig. 1B:: Schematische Darstellung zur Auswertung einer EKG-Kurve anhand der Herzratenvariabilität (HRV), die der Variabilität der RR-Strecke entspricht, des Winkels □, der die Steigung der R-Zacke wiedergibt, und der Fläche unter der EKG-Kurve (A im gestrichelten Bereich).
- Fig. 2:: Schematische Darstellung zu Platzierung der vier Elektroden zur Aufprägung der Frequenzen und elektrokardiographischen Messung am Oberkörper des Patienten.
- Fig. 3:: Darstellung einer EKG-Messreihe mit den erfassten EKG-Kurven in der oberen Reihe und den Abweichungen von Messwert und Standardwert in der unteren Reihe. Der umrahmte Bereich gibt eine Resonanzsituation wieder, bei der es durch die beiden aufgeprägten Frequenzen zu einer deutlichen positiven Differenz zwischen Messwert und Standardwert kommt.
- Fig. 4:: Darstellung von Resonanzfrequenzen einer Messung mit 100 Messwerten (X-Achse). Die y-Achse gibt die Abweichung vom Standardwert an. Stimulierende Resonanzfrequenzen sind als geschlossene Punkte, abschwächende Resonanzfrequenzen als offene Punkte dargestellt.
- Fig. 5A:: Darstellung der Resonanzfrequenzen einer Messung mit 200 Messwerten (von 00.00 bis 99.50) basierend auf der Testung von Patient A (siehe Beispiel 2). Die ermittelten 68 Resonanzfrequenzen sind grau unterlegt.
- Fig. 5B:: Darstellung der Resonanzfrequenzen eines in der Datenbank abgespeicherten Frequenzmusters für Gallenblasenentzündung mit insgesamt 52 Frequenzgrundwerten, die als umrandete Kästchen hervorgehoben sind.
- Fig. 6:: Ergebnis des Vergleichs der ermittelten Resonanzfrequenzen aus Figur 5A mit dem Frequenzmuster aus Figur 5B. Die übereinstimmenden Werte sind grau unterlegt und als umrandete Kästchen hervorgehoben.
- Fig. 7:: Darstellung der Resonanzfrequenzen einer Messung mit 200 Messwerten (von 00.00 bis 99.50) basierend auf der Testung von Patient B (siehe Beispiel 3). Die ermittelten 56 Resonanzfrequenzen sind mit ihrer jeweiligen Polarität aufgelistet. Stimulierende Sequenzen sind mit "R.+" gekennzeichnet, abschwächende Frequenzen mit "R.-".
- Fig. 8:: Ergebnis des Vergleichs der ermittelten Resonanzfrequenzen aus Figur 7 mit dem in der Datenbank abgespeicherten Frequenzmustern. Die relevanten Frequenzmuster sind unter Nr. 2 bis Nr. 5 aufgeführt. Frequenzmuster Nr. 1, Nr. 6 und Nr. 7 werden standardmäßig im Rahmen einer Therapie verwendet und sind deshalb mit aufgelistet.
- Fig. 9:: Schematische Darstellung eines Bioresonanzgerätes (zur näheren Beschreibung siehe Beispiel 4).
- Fig. 10:: Schematische Darstellung eines Regelkreises, wie er mit dem Verfahren verwirklicht werden kann, konkretisiert auf die Herzratenvariabilität, die anhand der EKG-Daten berechnet wird.

## Patentansprüche

1. Medizinisches Gerät zum Erfassen und Erzeugen von Resonanzfrequenzen umfassend die folgenden Module:
- ein Modul zur Aufnahme elektrophysiologischer Messwerte am menschlichen oder tierischen Patienten,
- ein Speichermodul umfassend eine Datenbank mit vorbekannten Resonanzfrequenzmustern,
- ein Modul zum Erzeugen elektromagnetischer Frequenzen, sowie
- mindestens eine Elektrode, die sowohl der Erfassung der elektrophysiologischen Messwerte als auch der Aufprägung der elektromagnetischen Frequenzen auf den Patienten dient,
wobei das Gerät für eine Steuerung umfassend die folgenden Schritte ausgestaltet ist:
a) Ermitteln von elektrophysiologischen Datenpunkten des Patienten in Abhängigkeit von auf den Patienten aufgeprägten elektromagnetischen Frequenzen,
b) Vergleichen dieser elektrophysiologischen Datenpunkte mit einem Standardwert für diese elektrophysiologischen Datenpunkte,
c) Identifizieren mindestens einer der Frequenzen aus Schritt a), bei der der elektrophysiologische Datenpunkt von dem Standardwert abweicht, als Resonanzfrequenz und
d) Abgleichen der mindestens einen Resonanzfrequenz des Patienten mit der Datenbank mit vorbekannten Resonanzfrequenzmustern.

## Claims

1. Medical device for detecting and generating resonance frequencies comprising the following modules:
- a module for recording electrophysiological measurements on human or animal patients,
- a storage module comprising a database with already known resonance frequency patterns,
- a module for generating electromagnetic frequencies and
- at least one electrode that is used both for detecting electrophysiological measurements and for imprinting the electromagnetic frequencies on the patient,
wherein the device is configured for a control comprising the following steps:
a) determining electrophysiological data points of the patient depending on electromagnetic frequencies imprinted on the patient,
b) comparing these electrophysiological data points with a standard value for these electrophysiological data points,
c) identifying at least one of the frequencies from step a), in which the electrophysiological data point deviates from the standard value, as a resonance frequency, and
d) matching the at least one resonance frequency of the patient with the database of already known resonance frequency patterns.

## Revendications

1. Dispositif médical pour l'enregistrement et la génération de fréquences de résonance comprenant les modules suivants :
- un module d'enregistrement des mesures électrophysiologiques sur un patient humain ou animal,
- un module de mémoire comprenant une base de données avec des modèles de fréquence de résonance connus au préalable,
- un module de génération de fréquences électromagnétiques, et
- au moins une électrode servant à la fois à l'enregistrement des mesures électrophysiologiques et à l'application des fréquences électromagnétiques sur le patient,
le dispositif étant configuré pour une commande comprenant les étapes suivantes :
a) Détermination des points de données électrophysiologiques du patient en fonction des fréquences électromagnétiques appliquées au patient,
b) Comparaison de ces points de données électrophysiologiques à une valeur standard pour ces points de données électrophysiologiques,
c) Identification d'au moins l'une des fréquences de l'étape a), à laquelle le point de données électrophysiologiques s'écarte de la valeur par défaut, comme étant la fréquence de résonance et
d) Mise en correspondance de l'au moins une fréquence de résonance du patient avec la base de données de modèles de fréquence de résonance connus au préalable.
